# EUROPEAN PATENT APPLICATION

(11) **EP 4 105 200 A1**
(43) Date of publication of application: **21.12.2022**
(21) Application number: 21754249.7
(22) Date of filing: 12.02.2021
(51) Int. Cl.: C07C 335/24, C07D 215/48, A61K 31/47

(54) **METHOD FOR PRODUCING ACYLTHIOUREA COMPOUND**

(30) Priority: 14.02.2020 JP 2020023754; 18.05.2020 JP 2020086940; 18.09.2020 JP 2020157815; 18.09.2020 JP 2020157816
(71) Applicant: Taiho Pharmaceutical Co., Ltd., Chiyoda-ku, Tokyo 101-8444 (JP)
(72) Inventor: SUDA Yoshimitsu, Tokyo 101-8444 (JP)
(74) Representative: Hoffmann Eitle
(86) International application number: PCT/JP2021/005272
(87) International publication number: WO 2021/162096

(57) **Abstract**

The present invention relates to a method for producing 4-(2-fluoro-4-(3-(2-phenylacetyl)thioureido)phenoxy)-7-methoxy-N-methylquinoline-6-carboxamide, the method comprising a step for linking 4-(4-amino-2-fluorophenoxy)-7-methoxy-N-methylquinoline-6-carboxamide and 2-phenylacetyl isothiocyanate.

## Description

### TECHNICAL FIELD

The present invention relates to a process for producing an acylthiourea compound, and more particularly to a process for producing an acylthiourea compound capable of mass production.

### BACKGROUND ART

When industrially producing active pharmaceutical ingredients (hereinafter, also referred to as API), a process of producing APIs from a raw material requires a small number of steps and a high yield. Further, since APIs are required to be mass-produced, operations that are not suitable for mass production cannot be adopted. The quality of APIs produced by such a process must match a standard or the like defined by an International Council for Harmonisation of Technical Requirements for Pharmaceuticals for Human Use (hereinafter, also referred to as ICH). Examples of a target to be the standard include an impurity, a residual solvent, a residual metal, and the like contained in the APIs.

Patent Literature 1 discloses that 4-(2-fluoro-4-(3-(2-phenylacetyl)thioureido)phenoxy)-7-methoxy-N-methylquinoline-6-carboxamide (hereinafter also referred to as compound 1) is a compound having an antitumor effect. Patent Literatures 2 and 3 disclose that compound 1 exhibits a therapeutic effect on osteoporosis and fibrosis. Patent Literature 4 discloses that when administering compound 1 to a human, compound 1 is administered after forming a mesylic acid salt. Patent Literature 5 discloses an example of a formulation of compound 1.

Patent Literature 1 describes that tert-butyl 4-chloro-7-methoxyquinoline-6-carboxylate is derived from 4-hydroxy-7-methoxyquinoline-6-carboxylic acid. Then, after introducing nitrophenol into the tert-butyl 4-chloro-7-methoxyquinoline-6-carboxylate, compound 1 is synthesized through steps of reducing a nitro group, derive acylthiourea from aniline, deprotecting carboxylic acid, and introducing methylamine in this order.

Further, according to Patent Literature 1, 4-(4-amino-2-fluorophenoxy)-7-methoxy-N-methylquinoline-6-carboxamide (hereinafter, also referred to as related substance 1) is described as one precursor of an acylthiourea derivative.

### CITATION LIST

### PATENT LITERATURE

Patent Literature 1: WO 2009/125597
Patent Literature 2: WO 2015/046484
Patent Literature 3: WO 2016/208744
Patent Literature 4: WO 2016/175305
Patent Literature 5: WO 2018/151177

### SUMMARY OF INVENTION

### TECHNICAL PROBLEM

When producing APIs, it is required that a producing process include steps by which an intermediate and an API can be obtained with a small number of steps and at a high yield. Furthermore, it is required these steps include only an easy operation and not include an operation that cannot be mass-produced. Moreover, it is required that the produced APIs match high quality as medicines.

On the other hand, according to Patent Literature 1, the production of compound 1 includes a step of protecting and deprotecting a carboxylic acid with tert-butyl, and a producing process with a smaller number of steps is required. However, a method for synthesizing compound 1, which does not include a step of protecting and deprotecting tert-butyl, has not been found. Furthermore, the step for producing compound 1 described in Patent Literature 1 includes purification by column chromatography, and is not suitable for mass production.

Patent Literature 1 describes 4-(4-amino-2-fluorophenoxy)-7-methoxy-N-methylquinoline-6-carboxamide (hereinafter also referred to as related substance 1), but there is no example in which compound 1 is directly derived from related substance 1 by introducing an acylthiourea group which is a partial structure of compound 1. In addition, it is not described that related substance 1 can be contained in the APIs.

In view of the above circumstances, an object of the present invention is to provide a process for producing an acylthiourea compound having a specific structure, which can be mass-produced in the production of a mesylic acid salt of compound 1 as an API.

### SOLUTION TO PROBLEM

As a result of intensive studies on the above problems, the present inventors have found a new process for producing compound 1, which is useful as an API capable of mass production. Further, the present inventors have found that a mesylic acid salt of compound 1, which is an API having a quality suitable for medicines, can be obtained by this producing process.

That is, the present invention provides the following [1] to [5].
[1] A process for producing 4-(2-fluoro-4- (3 -(2-phenylacetyl)thioureido)phenoxy)-7-methoxy-N-methylquinoline-6-carboxamide, including: coupling 4-(4-amino-2-fluorophenoxy)-7-methoxy-N-methylquinoline-6-carboxamide to 2-phenylacetyl isothiocyanate.
[2] The producing process according to [1], including: obtaining the 4-(4-amino-2-fluorophenoxy)-7-methoxy-N-methylquinoline-6-carboxamide from methyl 4-(4-amino-2-fluorophenoxy)-7-methoxyquinoline-6-carboxylate by aminolysis.
[3] The producing process according to [1] or [2], including: deriving the 2-phenylacetyl isothiocyanate from 2-phenylacetyl chloride.
[4] The producing process according to any one of [1] to [3], wherein a solvent which contains toluene and ethanol is used in the coupling step.
[5] A process for producing a mesylic acid salt of 4-(2-fluoro-4-(3-(2-phenylacetyl)thioureido)phenoxy)-7-methoxy-N-methylquinoline-6-carboxamide, including: producing the mesylic acid salt from 4-(2-fluoro-4-(3-(2-phenylacetyl)thioureido)phenoxy)-7-methoxy-N-methylquinoline-6-carboxamide obtained by the producing process according to any one of [1] to [4].

### ADVANTAGEOUS EFFECTS OF INVENTION

According to the present invention, a free form of compound 1 can be synthesized from related substance 1 in one step, and APIs having a quality suitable for medicines can be mass-produced.

### DESCRIPTION OF EMBODIMENTS

### «Process for Producing Compound 1»

One embodiment of the present invention relates to a process for producing 4-(2-fluoro-4-(3-(2-phenylacetyl)thioureido)phenoxy)-7-methoxy-N-methylquinoline-6-carboxamide from 4-(4-amino-2-fluorophenoxy)-7-methoxy-N-methylquinoline-6-carboxamide, and involves the following reaction. That is, compound 1 can be produced from related substance 1 by adopting a step of coupling 4-(4-amino-2-fluorophenoxy)-7-methoxy-N-methylquinoline-6-carboxamide to 2-phenylacetyl isothiocyanate. In structural formulae in the present specification, Me represents a methyl group.

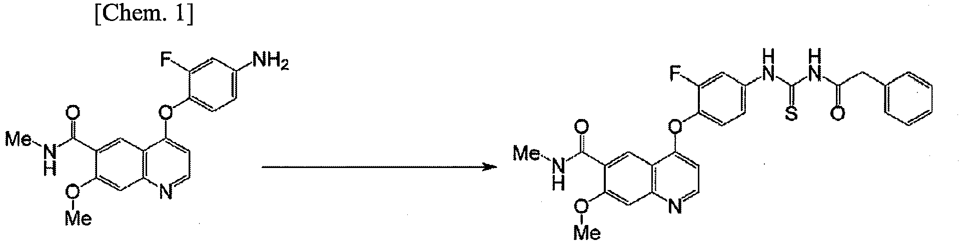

4-(2-fluoro-4-(3-(2-phenylacetyl) thioureido) phenoxy) -7-methoxy-N-methylquinoline-6-carboxamide produced in the present embodiment is an acylthiourea compound having the following structure, and is represented as "compound 1" in the present specification.

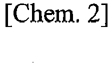

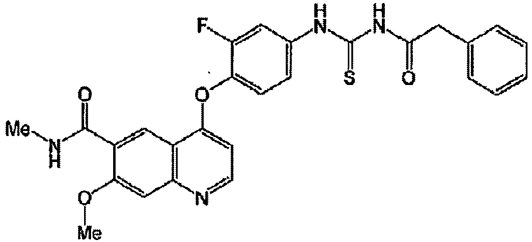

A free form of compound 1 in the present embodiment is a compound described in Patent Literature 1, and can be produced by a generally known method. Compound 1 or a pharmaceutically acceptable salt thereof is useful as an antitumor agent, an osteoporosis therapeutic agent, and a fibrosis therapeutic agent. Then, compound 1 is derived from a free form into a mesylic acid salt. The mesylic acid salt is used as an API. The API is not administered to a patient as it is. A preparation thereof is appropriately produced by a generally known method or the like, and then the preparation is administered to the patient.

Compound 1 in the present embodiment is preferably a salt when used as an API. A mesylic acid salt is preferred for the above reasons, but other salts are not excluded in any way.

Examples of the other salts include salts with inorganic bases, salts with organic bases, salts with inorganic acids, salts with organic acids, salts with acidic amino acids, and salts with basic amino acids as shown below.

Examples of the salts with the inorganic bases include alkali metal salts such as a sodium salt and a potassium salt, and alkaline earth metal salts such as a magnesium salt and a calcium salt.

Examples of the salts with the organic bases include trimethylamine, triethylamine, pyridine, N-methylpyridine, N-methylpyrrolidine, ethanolamine, diethanolamine, triethanolamine, and dicyclohexylamine.

Examples of the inorganic acids include hydrochloric acid, sulfuric acid, hydrobromic acid, hydroiodic acid, nitric acid, and phosphoric acid.

Examples of the organic acids include formic acid, acetic acid, propionic acid, oxalic acid, malonic acid, succinic acid, ascorbic acid, isoascorbic acid, mandelic acid, glutaric acid, adipic acid, fumaric acid, aspartic acid, maleic acid, lactic acid, malic acid, hippuric acid, citric acid, tartaric acid, carbonic acid, picric acid, mesylic acid, benzenesulfonic acid, and p-toluenesulfonic acid.

Examples of the acidic amino acids include glutamic acid and aspartic acid. Examples of the basic amino acids include lysine, asparagine, and ornithine.

4-(4-amino-2-fluorophenoxy)-7-methoxy-N-methylquinoline-6-carboxamide in the present embodiment is a compound having the following structure, and is referred to as "related substance 1" in the present specification.

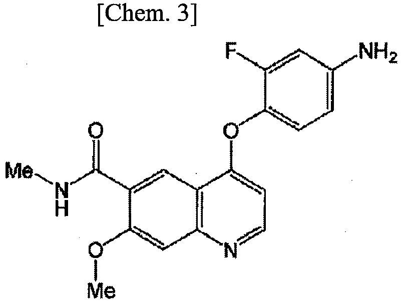

In the present embodiment, related substance 1 is a starting material from which compound 1 is derived by introducing acylthiourea.

A batch production, which is used in the production of a product or a compound, is a method in which a predetermined amount of products or compounds is produced at a time while a predetermined amount of raw materials is charged and each step is sequentially performed.

In the batch production, the amount of the raw materials charged in one production, which is called one batch is, for example, 1 kg or more, that is, the amount of the raw materials used in one production of a desired compound is, for example, 1 kg or more, which is called mass production in the present embodiment. The desired compound includes, in addition to the API, an intermediate to be used in the production of the API.

In the present embodiment, when producing compound 1 from related substance 1, a reagent to be used for introducing an acylthiourea group is not particularly limited as long as it is a commonly known reagent. Examples thereof include 2-phenylacetyl isothiocyanate. Among them, it is preferable to couple related substance 1 to 2-phenylacetylisothiocyanate.

2-phenylacetyl isothiocyanate has the following structure, and is preferably derived from 2-phenylacetyl chloride. Specifically, 2-phenylacetyl isothiocyanate can be produced by causing a reaction between 2-phenylacetyl chloride and potassium thiocyanate. In addition, 2-phenylacetyl isothiocyanate may be produced by another method, or may be a commercially available product.

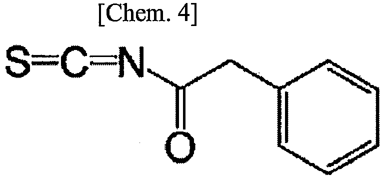

In the present embodiment, 2-phenylacetyl isothiocyanate can be used in an amount of 1.0 to 100 molar equivalents, preferably 1.2 molar equivalents or more, and particularly preferably 1.5 molar equivalents or more, with respect to related substance 1 as a starting material. The equivalent number is preferably 20 molar equivalents or less, more preferably 10 molar equivalents or less, even more preferably 5.0 molar equivalents or less, still more preferably 3.0 molar equivalents or less, yet still more preferably 2.5 molar equivalents or less, and particularly preferably 2.2 molar equivalents or less.

In the present embodiment, a reaction time is not particularly limited, and may be, for example, 0.5 hour to 500 hours, but is preferably 6 hours or more, and preferably 20 hours or less.

In the present embodiment, a reaction temperature is not particularly limited as long as it is equal to or lower than the boiling point of a solvent to be adopted, and is, for example, 0 to 100°C, but is preferably 15°C or higher, more preferably 20 °C or higher, and is preferably 50°C or lower, more preferably 30°C or lower.

In the present embodiment, the solvent to be adopted is not particularly limited as long as it is not a solvent capable of remarkably decomposing related substance 1, compound 1, and 2-phenylacetyl isothiocyanate. Examples thereof include water, C5-C10 hydrocarbons, C6-C14 aromatic hydrocarbons, C1-C6 alcohols, C3-C10 aliphatic carboxylic acid esters, C3-C10 ketones, C4-C10 ethers, C3-C5 aprotic polar organic solvents, and mixed solvents thereof. In the present specification, the description represented by the number C means the number of carbon atoms, and means the total number of carbon atoms contained in such a compound.

The C5-C10 hydrocarbons are hydrocarbons having 5 to 10 carbon atoms. Examples thereof include pentane, hexane, heptane, octane, nonane, decane, cyclopentane, cyclohexane, cycloheptane, cyclooctane, cyclononane, and cyclodecane.

The C6-C14 aromatic hydrocarbons are aromatic hydrocarbons having 6 to 14 carbon atoms. Examples thereof include benzene, naphthalene, anthracene, toluene, xylene, cumene, styrene, and phenanthrene.

The C1-C6 alcohols are alcohols having 1 to 6 carbon atoms. Examples thereof include methanol, ethanol, n-propanol, isopropanol, n-butanol, tert-butanol, n-pentanol and n-hexanol.

The C3-C10 aliphatic carboxylic acid esters are aliphatic carboxylic acid esters having 3 to 10 carbon atoms. Examples thereof include propyl formate, methyl acetate, ethyl acetate, propyl acetate, methyl propionate, ethyl propionate, propyl propionate, methyl butanoate, and ethyl butanoate.

The C3-C10 ketones are ketones having 3 to 10 carbon atoms. Examples thereof include acetone, ethyl methyl ketone, diethyl ketone, isopropyl methyl ketone, and cyclohexanone.

The C4-C10 ethers are ethers having 4 to 10 carbon atoms. Examples thereof include diethyl ether, tert-butyl methyl ether, tetrahydrofuran, and 1,4-dioxane.

The C3-C5 aprotic polar organic solvents are aprotic polar organic solvents having 3 to 5 carbon atoms. Examples thereof include N,N-dimethylformamide, N,N-dimethylacetamide, and N-methylpyrrolidone.

In the present embodiment, the solvent to be adopted is preferably C6-C14 aromatic hydrocarbons, C1-C6 alcohols, or a mixed solvent thereof, and particularly preferably toluene, ethanol, or a mixed solvent thereof.

When a solvent (mixed solvent) which contains toluene and ethanol is adopted as the solvent in the present embodiment, the amount of toluene may be 0.01 to 100 times the amount of ethanol in terms of a volume ratio, but is preferably 0.1 times or more, more preferably 0.5 times or more, even more preferably 1.0 times or more, still more preferably 2.0 times or more, yet still more preferably 3.0 times or more, and particularly preferably 4.0 times or more. The amount of toluene is preferably 50 times or less, more preferably 25 times or less, even more preferably 10 times or less, still more preferably 8.0 times or less, yet still more preferably 7.0 times or less, and particularly preferably 6.0 times or less, the amount of ethanol in terms of a volume ratio.

A process for producing compound 1 according to the present embodiment is characterized by including a step of coupling related substance 1 to 2-phenylacetyl isothiocyanate.

Preferably, the process for producing compound 1 being characterized by including a step of coupling related substance 1 to 2-phenylacetyl isothiocyanate, includes a step of deriving the 2-phenylacetyl isothiocyanate from 2-phenylacetyl chloride.

More preferably, the process for producing compound 1 being characterized by including a step of coupling related substance 1 to 2-phenylacetyl isothiocyanate in a solvent which contains toluene and ethanol, includes a step of deriving the 2-phenylacetyl isothiocyanate from 2-phenylacetyl chloride.

Even more preferably, the process for producing compound 1 being characterized by including a step of coupling related substance 1 to 2-phenylacetyl isothiocyanate in a solvent in which toluene and ethanol are contained and the amount of toluene is 0.01 to 100 times the amount of ethanol in terms of a volume ratio, includes a step of deriving the 2-phenylacetyl isothiocyanate from 2-phenylacetyl chloride.

Particularly preferably, the process for producing compound 1 being characterized by including a step of coupling related substance 1 to 2-phenylacetyl isothiocyanate in a solvent in which toluene and ethanol are contained and the amount of toluene is 0.01 to 100 times the amount of ethanol in terms of a volume ratio, in which the amount of the 2-phenylacetyl isothiocyanate used in this step is 1.0 to 5.0 molar equivalents of related substance 1, includes a step of deriving the 2-phenylacetyl isothiocyanate from 2-phenylacetyl chloride.

In the process for producing compound 1 according to the present embodiment, related substance 1 is preferably obtained by aminolysis of methyl 4-(4-amino-2-fluorophenoxy)-7-methoxyquinoline-6-carboxylate. That is, the process for producing compound 1 according to the present embodiment preferably further includes a step of obtaining related substance 1 from methyl 4-(4-amino-2-fluorophenoxy)-7-methoxyquinoline-6-carboxylate by aminolysis. Aminolysis is one of methods for constructing an amide from an ester, and includes a step of acting an ester with an amine.

In the production of related substance 1, in addition to methyl 4-(4-amino-2-fluorophenoxy)-7-methoxyquinoline-6-carboxylate, 4-(4-nitro-2-fluorophenoxy)-7-methoxy-N-methylquinoline-6-carboxamide is considered as a raw material thereof.

However, as a result of studies by the present inventors, it was suggested that elimination of fluoronitrophenol was caused by aminolysis of methyl 4-(4-nitro-2-fluorophenoxy)-7-methoxyquinoline-6-carboxylate in order to obtain 4-(4-nitro-2-fluorophenoxy)-7-methoxy-N-methylquinoline-6-carboxamide. As a result, it was suggested that a route for producing compound 1 by obtaining related substance 1 from 4-(4-nitro-2-fluorophenoxy)-7-methoxy-N-methylquinoline-6-carboxamide is not suitable for mass production because a yield is low.

In addition to the above, the present inventors also studied a route for obtaining compound 1 by first deriving acylthiourea from an amino group of methyl 4-(4-amino-2-fluorophenoxy)-7-methoxyquinoline-6-carboxylate to synthesize methyl 4-(2-fluoro-4-(3-(2-phenylacetyl)thioureido)phenoxy)-7-methoxyquinoline-6-carboxylate, and then performing aminolysis. However, an acylthiourea moiety was decomposed in the aminolysis, and compound 1 could not be obtained at a high yield.

On the other hand, it was found that when methyl 4-(4-amino-2-fluorophenoxy)-7-methoxyquinoline-6-carboxylate was subjected to aminolysis, the elimination of fluoronitrophenol as described above did not occur, and related substance 1 was obtained at a high yield.

Therefore, the producing process according to the present embodiment is characterized by obtaining compound 1 from related substance 1, but is preferably a method in which compound 1 is obtained from related substance 1, and the related substance 1 is obtained from methyl 4-(4-amino-2-fluorophenoxy)-7-methoxyquinoline-6-carboxylate. The producing process according to the present embodiment is more preferably a method in which compound 1 is obtained from related substance 1, the related substance 1 is obtained from methyl 4-(4-amino-2-fluorophenoxy)-7-methoxyquinoline-6-carboxylate, and the methyl 4-(4-amino-2-fluorophenoxy)-7-methoxyquinoline-6-carboxylate is obtained from methyl 4-(4-nitro-2-fluorophenoxy)-7-methoxyquinoline-6-carboxylate.

Here, the aminolysis can be performed under known conditions. For example, in a method of reacting methylamine at a high concentration in a mixed solvent of N-methyl-2-pyrrolidone and water, related substance 1 was obtained in a yield of 80% or more.

Further, methyl 4-(4-amino-2-fluorophenoxy)-7-methoxyquinoline-6-carboxylate is obtained by reducing a nitro group of methyl 4-(4-nitro-2-fluorophenoxy)-7-methoxyquinoline-6-carboxylate. This reduction reaction can also be performed under known conditions. For example, in a method using iron and a mixed solvent of hydrochloric acid and methanol, a reductant was obtained in a yield of 90% to 98%. For example, in the case of catalytic reduction using carbon-supported platinum and hydrogen, a reductant was obtained at a yield of approximately 100%.

From the above results, it was found that when producing compound 1, it is preferable to include a step of obtaining related substance 1 from methyl 4-(4-amino-2-fluorophenoxy)-7-methoxyquinoline-6-carboxylate by aminolysis.

When mass-producing compound 1, the producing process including the step of coupling 2-phenylacetyl isothiocyanate to related substance 1 is preferable because the number of steps is small. Furthermore, it can be said that column chromatography is not necessary, which is also suitable for mass production. In addition, the producing process is suitable for mass production from the viewpoints that the yield and quality reproducibility of the obtained compound 1 are good, production is possible even when the stirring ability during the reaction is lowered as compared with the case of small amount synthesis, production is possible even when a time required for charging the raw material and the reagent is longer as compared with the case of small amount synthesis, a production cost can be suppressed, and the like.

### «Process for Producing Mesylic Acid Salt of Compound 1»

Compound 1 obtained in this manner is purified as necessary, and then mesylic acid salt is derived from the compound 1 by the method described in Patent Literature 4 or the like. The mesylic acid salt is used as an API.

A solvent used when deriving a mesylic acid salt of compound 1 from compound 1 is not particularly limited, but preferably includes water, alcohols, aliphatic carboxylic acid esters, ketones, ethers, hydrocarbons, and aprotic polar solvent. Also usable is a mixed solvent composed of two or more of these.

Examples of the alcohols include methanol, ethanol, n-propanol, and isopropanol. Preferred are ethanol and isopropanol.

Examples of the aliphatic carboxylic acid esters include methyl formate, ethyl formate, methyl acetate, ethyl acetate, isopropyl acetate, and butyl acetate. Preferred is ethyl acetate.

Examples of the ketones include acetone, methyl ethyl ketone, methyl isopropyl ketone, methyl isobutyl ketone, and cyclohexanone. Preferred is acetone, methyl ethyl ketone, or methyl isobutyl ketone.

Examples of the ethers include diethyl ether, tert-butyl methyl ether, tetrahydrofuran, and 1,4-dioxane.

Examples of the hydrocarbons include n-hexane, n-pentane, n-heptane, cyclohexane, cyclopentane, and petroleum ether.

Examples of the aprotic polar solvents include acetonitrile, N-methyl-2-pyrrolidone, N,N-dimethylformamide, N,N-dimethylacetamide, and dimethyl sulfoxide.

The solvent is preferably water, alcohols, aliphatic carboxylic acid esters, ketones, or mixed solvents each composed of two or more of these, and more preferably ethanol, isopropanol, ethyl acetate, acetone, methyl ethyl ketone, methyl isobutyl ketone, or an acetone/water mixed solvent. An acetone/water mixed solvent is particularly preferable.

With respect to the ratio between the solvents in the acetone/water mixed solvent, the amount of the acetone per part by volume of the water is preferably 0.1-100 parts by volume, more preferably 0.5 part by volume or more, even more preferably 1 part by volume or more, still more preferably 5 parts by volume or more, and more preferably 20 parts by volume or less, even more preferably 10 parts by volume or less, and still more preferably 5 parts by volume or less.

The amount of the solvent that can be used in the process for producing the mesylic acid salt of the present embodiment is 0.1-100 (volume/weight) times the amount of compound 1. The amount thereof is preferably 1-50 (volume/weight) times, more preferably 5-30 (volume/weight) times.

A temperature in the step for precipitating the mesylic acid salt of the present embodiment is suitably set in accordance with the solvent to be used, in the range of 0°C to the boiling point of the solvent. The temperature in the step for precipitation need not be constant, and the mixture can be heated or cooled at temperatures between 0°C and the boiling point of the solvent.

The stirring in the step for precipitating the mesylic acid salt of the present embodiment is conducted by suitably using a stirring machine, stirring blades, magnetic stirrer, or the like in accordance with the solvent amount and the size of the reaction tank. The stirring speed is 1-600 rpm, preferably 10-300 rpm.

In general, in case where the period of stirring for precipitating salts is too short, the precipitation does not proceed sufficiently, making it impossible to obtain salts or crystals in high yield. Meanwhile, in case where the period thereof is too long, the active ingredients suffer decomposition, resulting in a decrease in yield. An appropriate period is hence set. The stirring period in the step for precipitating the mesylic acid salt of the present embodiment is, for example, 1 minute to 120 hours, and is preferably 1-72 hours, more preferably 3-48 hours.

In the step for precipitating the mesylic acid salt of the present embodiment, the mesylic acid salt of compound 1 may be added as seed crystal. The amount of the seed crystal to be added is 0.1-10% by weight, preferably 1-3% by weight, of a theoretical yield of the mesylic acid salt of compound 1 in the crystallization.

The mesylic acid salt of the present embodiment which has precipitated in the solvent can be isolated and purified by known separation/purification means, such as filtration, cleaning with an organic solvent, and vacuum drying. Examples of the organic solvent for use in the cleaning include the same solvents as those usable for the production.

### <<Method for Purifying Compound 1>>

In the present invention, in order to improve the chemical purity of compound 1, purification may be performed in each step in the production of compound 1, or purification may be performed on the produced compound 1. The purification is not particularly limited, and various purification methods used in the field can be used. In one embodiment of the present invention, specifically, a purification step of removing related substances from a free form of compound 1 is performed.

In the present embodiment, the free form of compound 1 used for purification may be produced by a generally known method, or may be derived from related substance 1 and 2-phenylacetyl isothiocyanate, but is preferably derived from related substance 1 and 2-phenylacetyl isothiocyanate. The expression "derived from related substance 1 and phenylacetyl isothiocyanate" has the same meaning as that obtained by the step of coupling related substance 1 to phenylacetyl isothiocyanate described in «Process for Producing Compound 1» above, and the preferred embodiment thereof is also the same as the preferred embodiment described above.

In the present embodiment, the purpose of the purification step is to improve the chemical purity of compound 1 after purification. The chemical purity herein refers to a purity calculated by high performance liquid chromatography.

In the present embodiment, the purification step includes a step of using a method such as recrystallization or heat suspension, and preferably includes a step of recrystallization.

In the present embodiment, the impurity to be removed by purification is not particularly limited as long as it is a compound other than compound 1 or the like, but is preferably related substances 1 to 5 and salts thereof, and more preferably related substances 1 to 3 and 5 and salts thereof.

In the present embodiment, a solvent to be used for purification is not particularly limited, but a solvent which contains N,N-dimethylacetamide (hereinafter, also referred to as DMA) and a protic polar solvent is preferable. Examples of the protic polar solvent include methanol, ethanol, and propanol. The solvent is more preferably a solvent which contains DMA and ethanol, or a solvent which contains DMA and 2-propanol, and still more preferably a solvent which contains DMA and ethanol. When a solvent which contains DMA and a protic polar solvent is used, DMA and the protic polar solvent may be pre-mixed to make a mixed solvent, or DMA and the protic polar solvent may be mixed as a result of using DMA and a protic polar solvent in sequence.

In the present embodiment, the amount of the solvent to be used for purification refers to the amount of the solvent in which compound 1 is dispersed when compound 1 is collected by filtration, and does not include the solvent to be used for cleaning after filtration. The amount of the solvent to be used for purification is not particularly limited as long as purification can be performed, but is preferably 0.5 to 100 (volume/weight) times, more preferably 2 to 50 (volume/weight) times, and still more preferably 5 to 20 (volume/weight) times the amount of compound 1 to be purified.

In the present embodiment, when recrystallization is adopted for purification, compound 1 is required to be dissolved in a solvent. The solvent to be adopted at this time is not particularly limited, but a solvent which contains only DMA or a solvent which contains DMA and a protic polar solvent is preferable, a solvent which contains DMA and a protic polar solvent is more preferable, and a solvent containing DMA and ethanol is even more preferable. A mixed solvent in which DMA and a protic polar solvent are pre-mixed may be used, or a protic polar solvent may be mixed after compound 1 is dissolved in DMA, resulting in a solvent mixture. Among them, from the viewpoint of obtaining compound 1 with high purity, it is preferable that a step of charging a protic polar solvent is performed after a step of dissolving compound 1 in DMA, and it is more preferable that a step of charging ethanol is performed after a step of dissolving compound 1 in DMA.

The present inventors have studied various solvents other than DMA upon recrystallization. For example, compound 1 may be dissolved in dimethyl sulfoxide (DMSO). However, it was suggested that when heating is performed for dissolution using DMSO, a thioketone (C=S) moiety in an acylthiourea structure of compound 1 became a ketone (C=O), and the structure was broken.

On the other hand, it was found that DMA can be satisfactorily heated and dissolved while maintaining the structure of compound 1, suggesting that DMA is preferable as a solvent used in the dissolution step.

In the present embodiment, when recrystallization is adopted for purification, a temperature at which compound 1 is dissolved in the solvent is not particularly limited, but is preferably 40-100°C, and more preferably 50-80°C. In the case of dissolving in DMA, the temperature for dissolution is more preferably 70-80°C.

In the present embodiment, the temperature at which the dissolved compound 1 is precipitated after dissolving compound 1 in a solvent is not particularly limited. For example, when charging ethanol to precipitate compound 1, the temperature at which ethanol is charged is preferably 50-80°C. Thereafter, the temperature for precipitation (crystallization) is preferably 45-55°C.

In the present embodiment, when recrystallization is adopted for purification, a time from when compound 1 is dissolved in a solvent to when compound 1 is precipitated and collected by filtration is not particularly limited as long as the precipitation amount of compound 1 is constant, but is preferably 1 hour or more, more preferably 1 to 100 hours, and still more preferably 1 to 72 hours.

In the present embodiment, when recrystallization is adopted for purification, a temperature at which compound 1 is collected by filtration is not particularly limited as long as the temperature is equal to or higher than the melting point of the solvent to be used, but is preferably -10 to 50°C, and more preferably 0-30°C.

In the present embodiment, when a solvent which contains DMA and ethanol is used as the solvent for recrystallization, a mixing ratio (volume ratio) of DMA to ethanol is preferably DMA: ethanol = 1:0.01 to 1:100, more preferably 1:0.01 to 1:10 or 1:0.1 to 1:100, even more preferably 1:0.1 to 1:10, and even more preferably 1:1 to 1:10.

The recrystallization step includes a step of dissolving compound 1 in a solvent and a step of crystallizing (precipitating) the dissolved compound 1.

In the present embodiment, when a solvent which contains DMA and ethanol is used as the solvent for recrystallization, DMA or a mixed solvent of DMA and a protic polar solvent is preferably used as the solvent to be used in the step of dissolving a free form of compound 1, and DMA is more preferably used. In order to dissolve the free form of compound 1, the amount of the solvent and the temperature can be set as described above.

Next, in the step of precipitating the dissolved compound 1, after dissolving compound 1 in the solvent described above, the free form of compound 1 can be precipitated by, for example, dropping a protic polar solvent, or by lowering the temperature with or instead thereof. The protic polar solvent is preferably ethanol.

Whether the free form of compound 1 thus obtained sufficiently satisfies the quality of the API is determined by analyzing the mesylic acid salt of compound 1. The determination is made in accordance with the standards described in the ICH, and the chemical purity serving as the standard at that time is calculated by measurement by high performance liquid chromatography or the like.

Therefore, the step of purifying compound 1 in the present embodiment relates particularly to a purification process of removing related substances from a free form of compound 1. The step of purifying compound 1 in the present embodiment is preferably a purification process of removing related substances from a free form of compound 1, and more preferably a purification process including a step of recrystallization.

The step of purifying compound 1 in the present embodiment is even more preferably a purification process of removing related substances from a free form of compound 1, and a purification process including a step of recrystallization using a solvent which contains N,N-dimethylacetamide and ethanol as a solvent.

In another embodiment, the purification step of removing related substances from a free form of compound 1 is more preferably a purification process including a step of dissolving compound 1 in DMA at 75 ± 5°C (70-80°C), then a step of charging ethanol at 65 ± 15°C (50-80°C), and a step of crystallizing thereafter at 50 ± 5°C (45 to 55°C).

The purification step of removing related substances from a free form of compound 1 is even more preferably a purification process of removing related substances from a free form of compound 1, and a purification process including a step of dissolving compound 1 in DMA having a volume ratio of DMA: ethanol of 1:1 to 1:100 to 1:100 to 1 at 75 ± 5°C (70-80°C), then a step of charging ethanol at 65 ± 15°C (50-80°C), and a step of crystallizing thereafter at 50 ± 5°C (45-55°C).

### «Related Substance of Compound 1»

When the produced compound 1 is decomposed, compounds such as related substance 1 and related substances 2 and 3 shown below are generated. Compound 1 produced by a process including a specific step may contain the following related substances 4 and 5.

Related substance 2 is N-((3-fluoro-4-hydroxyphenyl) carbamothioyl)-2-phenylacetamide and has the following structure.

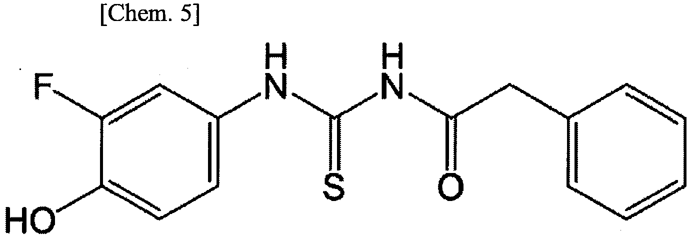

Related substance 3 is 4-hydroxy-7-methoxy-N-methylquinoline-6-carboxamide and has the following structure.

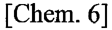

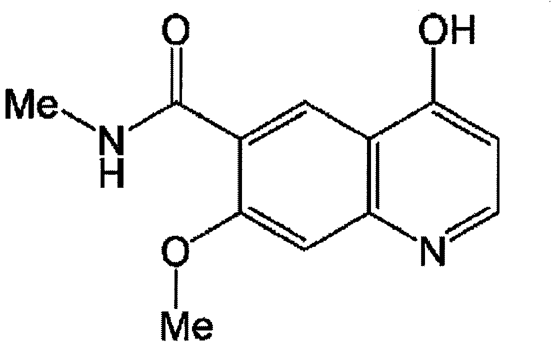

Related substance 4 is 7-methoxy-N-methyl-4-(4-(3-(2-phenylacetyl)thioureido)phenoxy)quinoline-6-carboxamide, and has the following structure.

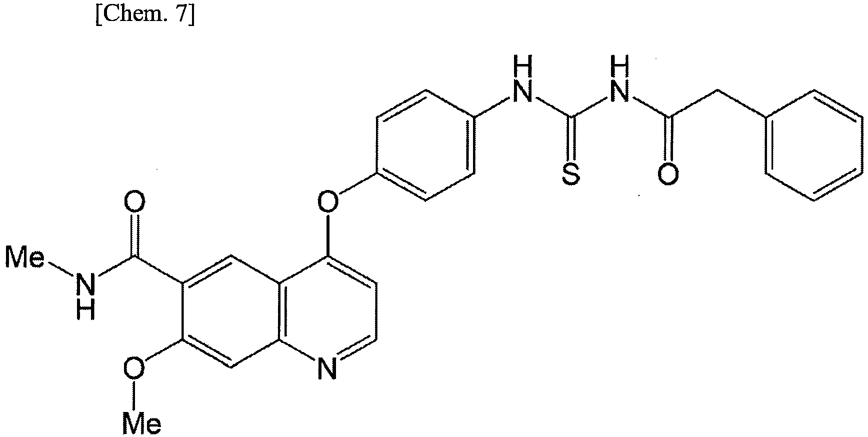

Related substance 5 is 4-(2-fluoro-4-(2-phenylacetamide)phenoxy)-7-methoxy-N-methylquinoline-6-carboxamide, and has the following structure.

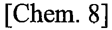

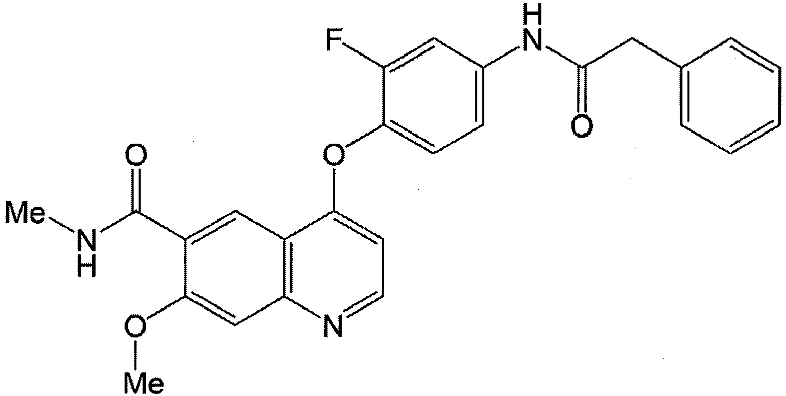

As described above, all of related substances 1 to 3 are compounds produced by decomposition of compound 1. Therefore, it can be said that related substances 1 to 3 and salts thereof are impurities that can be contained in the API and a preparation of compound 1.

Related substance 4 is a compound that can be contained in compound 1 produced by a process including the following steps.

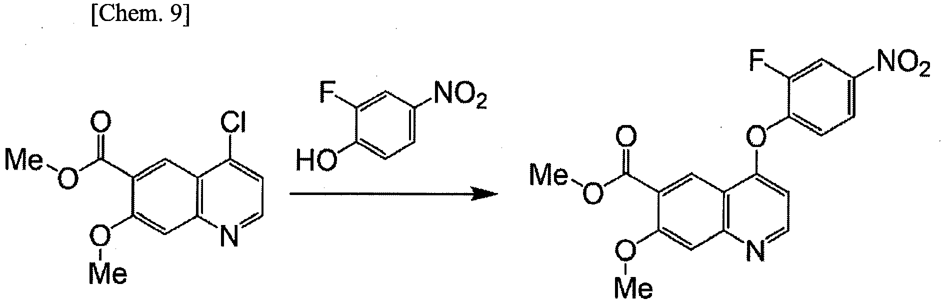

That is, related substance 4 is a compound derived from 4-nitrophenol or the like contained in 2-fluoro-4-nitrophenol in the coupling of methyl 4-chloro-7-methoxyquinoline-6-carboxylate and 2-fluoro-4-nitrophenol (described in Example 39a of Patent Literature 1), which is a step of obtaining methyl 4-(4-nitro-2-fluorophenoxy)-7-methoxyquinoline-6-carboxylate.

Related substance 5 is a by-product of compound 1 obtained in the step of coupling related substance 1 to 2-phenylacetyl isothiocyanate.

Both of related substances 4 and 5 are compounds produced in the production process of compound 1. Therefore, it can be said that related substances 4 and 5 and salts thereof are impurities that can be contained in the API and the preparation of compound 1.

Related substances 1 to 5 or salts thereof may be solvates (for example, hydrates) or non-solvates, and in the present embodiment, all of them are included in the "compound or a salt thereof". Related substances 1 to 5 also include tautomers thereof.

The salt of the compound is not particularly limited. Examples thereof include, in addition to the mesylic acid salt, addition salts with inorganic acids such as hydrochloric acid, hydrobromic acid, hydroiodic acid, hydrofluoric acid, and sulfuric acid, or an organic acid such as alkyl sulfuric acid such as methanesulfonic acid, p-toluenesulfonic acid, and benzenesulfonic acid, acetic acid, citric acid, tartaric acid, and maleic acid, salts with an alkali metal such as potassium and sodium, salts with an alkaline earth metal such as calcium and magnesium, and salts with an organic base such as ammonium salt, ethylamine salt, and arginine salt.

In the present specification, the descriptions of "related substance 1", "related substance 2", "related substance 3", "related substance 4", and "related substance 5" may be intended to include "salts" and "solvates" of the related substances.

A combination of related substances 1 to 5 described above or salts thereof may also be contained in the API and the preparation of compound 1.

The combination contains two or more of related substances 1 to 5 or salts thereof. A case where all of the combinations are two or more of related substances 1 to 5, a case where some of the combinations are one or more of related substances 1 to 5 and the remaining combinations are one or more salts of related substances 1 to 5, and a case where all of the combinations are two or more salts of related substances 1 to 5 are included. The combination may contain one of related substances 1 to 5 and a salt of the related substances 1 to 5.

The present invention includes compound 1 or a salt thereof as an API, in which a content of each of related substances 1 to 5 and salts thereof is less than 0.2% by mass of total API.

Examples of the process for producing compound 1 or a salt thereof, in which a content of each of related substances 1 to 5 and salts thereof is less than 0.2% by mass of total API, include a process for producing compound 1 or a salt thereof, which is derived from methyl 4-chloro-7-methoxyquinoline-6-carboxylate, 2-fluoro-4-nitrophenol, and 2-phenylacetyl isothiocyanate described above. In the case of being come from a starting material, a reagent, a solvent, or the like, the content of each of related substances 1 to 5 and salts thereof can be made less than 0.2% by mass of total API by controlling the starting material or the like used in the producing process. The content of each of related substances 1 to 5 and salts thereof can be determined by, for example, the method for analyzing related substances described above.

The above embodiment is preferably compound 1 or a salt thereof in which a content of each of related substances 1 to 5 and salts thereof is less than 0.2% by mass of total API, the compound 1 or a salt thereof which is derived from methyl 4-chloro-7-methoxyquinoline-6-carboxylate, 2-fluoro-4-nitrophenol, and 2-phenylacetyl isothiocyanate.

The above embodiment is more preferably compound 1 or a salt thereof in which a content of each of related substances 1 to 5 and salts thereof is less than 0.2% by mass of total API, the compound 1 or a salt thereof which is produced by a producing process including a step of coupling related substance 1 to 2-phenylacetyl isothiocyanate.

The above embodiment is even more preferably a mesylic acid salt of compound 1 in which a content of each of related substances 1 to 5 and salts thereof is less than 0.2% by mass of total API, the mesylic acid salt of compound 1 which is produced by a producing process in which a step of coupling related substance 1 to 2-phenylacetyl isothiocyanate is included and a content of related substance 1 charged in one production is 1 kg or more.

In another embodiment, the present invention is compound 1 or a salt thereof, in which a content of each of related substance 1 and a salt thereof is less than 0.2% by mass of total API. The present invention is preferably compound 1 or a salt thereof in which a content of each of related substance 1 and a salt thereof is less than 0.2% by mass of total API, the compound 1 or a salt thereof which is derived from methyl 4-chloro-7-methoxyquinoline-6-carboxylate, 2-fluoro-4-nitrophenol, and 2-phenylacetyl isothiocyanate. The present invention is more preferably compound 1 or a salt thereof in which a content of each of related substance 1 and a salt thereof is less than 0.2% by mass of total API, the compound 1 or a salt thereof which is produced by a producing process including a step of coupling related substance 1 to 2-phenylacetyl isothiocyanate. The present invention is even more preferably a mesylic acid salt of compound 1 in which a content of each of related substance 1 and a salt thereof is less than 0.2% by mass of total API, the mesylic acid salt of compound 1 which is produced by a producing process in which a step of coupling related substance 1 to 2-phenylacetyl isothiocyanate is included and a content of related substance 1 charged in one production is 1 kg or more.

In another embodiment, the present invention is compound 1 or a salt thereof, in which a content of each of related substance 2 and a salt thereof is less than 0.2% by mass of total API. The present invention is preferably compound 1 or a salt thereof in which a content of each of related substance 2 and a salt thereof is less than 0.2% by mass of total API, the compound 1 or a salt thereof which is derived from methyl 4-chloro-7-methoxyquinoline-6-carboxylate, 2-fluoro-4-nitrophenol, and 2-phenylacetyl isothiocyanate. The present invention is more preferably compound 1 or a salt thereof in which a content of each of related substance 2 and a salt thereof is less than 0.2% by mass of total API, the compound 1 or a salt thereof which is produced by a producing process including a step of coupling related substance 1 to 2-phenylacetyl isothiocyanate. The present invention is even more preferably a mesylic acid salt of compound 1 in which a content of each of related substance 2 and a salt thereof is less than 0.2% by mass of total API, the mesylic acid salt of compound 1 which is produced by a producing process in which a step of coupling related substance 1 to 2-phenylacetyl isothiocyanate is included and a content of related substance 1 charged in one production is 1 kg or more.

In another embodiment, the present invention is compound 1 or a salt thereof, in which a content of each of related substance 3 and a salt thereof is less than 0.2% by mass of total API. The present invention is preferably compound 1 or a salt thereof in which a content of each of related substance 3 and a salt thereof is less than 0.2% by mass of total API, the compound 1 or a salt thereof which is induced from methyl 4-chloro-7-methoxyquinoline-6-carboxylate, 2-fluoro-4-nitrophenol, and 2-phenylacetyl isothiocyanate. The present invention is more preferably compound 1 or a salt thereof in which a content of each of related substance 3 and a salt thereof is less than 0.2% by mass of total API, the compound 1 or a salt thereof which is produced by a producing process including a step of coupling related substance 1 to 2-phenylacetyl isothiocyanate. The present invention is even more preferably a mesylic acid salt of compound 1 in which a content of each of related substance 3 and a salt thereof is less than 0.2% by mass of total API, the mesylic acid salt of compound 1 which is produced by a producing process in which a step of coupling related substance 1 to 2-phenylacetyl isothiocyanate is included and a content of related substance 1 charged in one production is 1 kg or more.

In another embodiment, the present invention is compound 1 or a salt thereof, in which a content of each of related substance 4 and a salt thereof is less than 0.2% by mass of total API. The present invention is preferably compound 1 or a salt thereof in which a content of each of related substance 4 and a salt thereof is less than 0.2% by mass of total API, the compound 1 or a salt thereof which is derived from methyl 4-chloro-7-methoxyquinoline-6-carboxylate, 2-fluoro-4-nitrophenol, and 2-phenylacetyl isothiocyanate. The present invention is more preferably compound 1 or a salt thereof in which a content of each of related substance 4 and a salt thereof is less than 0.2% by mass of total API, the compound 1 or a salt thereof which is produced by a producing process including a step of coupling related substance 1 to 2-phenylacetyl isothiocyanate. The present invention is even more preferably a mesylic acid salt of compound 1 in which a content of each of related substance 4 and a salt thereof is less than 0.2% by mass of total API, the mesylic acid salt of compound 1 which is produced by a producing process in which a step of coupling related substance 1 to 2-phenylacetyl isothiocyanate is included and a content of related substance 1 charged in one production is 1 kg or more.

In another embodiment, the present invention is compound 1 or a salt thereof, in which a content of each of related substance 5 and a salt thereof is less than 0.2% by mass of total API. The present invention is preferably compound 1 or a salt thereof in which a content of each of related substances 5 and a salt thereof is less than 0.2% by mass of total API, the compound 1 or a salt thereof which is derived from methyl 4-chloro-7-methoxyquinoline-6-carboxylate, 2-fluoro-4-nitrophenol, and 2-phenylacetyl isothiocyanate. The present invention is more preferably compound 1 or a salt thereof in which a content of each of related substance 5 and a salt thereof is less than 0.2% by mass of total API, the compound 1 or a salt thereof which is produced by a producing process including a step of coupling related substance 1 to 2-phenylacetyl isothiocyanate. The present invention is even more preferably a mesylic acid salt of compound 1 in which a content of each of related substance 5 and a salt thereof is less than 0.2% by mass of total API, the mesylic acid salt of compound 1 which is produced by a producing process in which a step of coupling related substance 1 to 2-phenylacetyl isothiocyanate is included and a content of related substance 1 charged in one production is 1 kg or more.

In another embodiment, the present invention is a process for producing compound 1 or a salt thereof, in which a content of each of related substances 1 to 5 and salts thereof is less than 0.2% by mass of total API. The present invention is preferably a process for producing compound 1 or a salt thereof in which a content of each of related substances 1 to 5 and salts thereof is less than 0.2% by mass of total API, the compound 1 or a salt thereof which is derived from methyl 4-chloro-7-methoxyquinoline-6-carboxylate, 2-fluoro-4-nitrophenol, and 2-phenylacetyl isothiocyanate. The present invention is more preferably a process for producing a mesylic acid salt of compound 1 in which a content of each of related substances 1 to 5 and salts thereof is less than 0.2% by mass of total API, the process which includes a step of coupling related substance 1 to 2-phenylacetyl isothiocyanate. The present invention is even more preferably a process for producing a mesylic acid salt of compound 1 in which a content of each of related substances 1 to 5 and salts thereof is less than 0.2% by mass of total API, the process in which a step of coupling related substance 1 to 2-phenylacetyl isothiocyanate is included and a content of related substance 1 charged in one production is 1 kg or more.

In another embodiment, the present invention is a process for producing compound 1 or a salt thereof, in which a content of each of related substance 1 and a salt thereof is less than 0.2% by mass of total API. The present invention is preferably a process for producing compound 1 or a salt thereof in which a content of each of related substance 1 and a salt thereof is less than 0.2% by mass of total API, the compound 1 or a salt thereof which is derived from methyl 4-chloro-7-methoxyquinoline-6-carboxylate, 2-fluoro-4-nitrophenol, and 2-phenylacetyl isothiocyanate. The present invention is more preferably a process for producing a mesylic acid salt of compound 1 in which a content of each of related substance 1 and a salt thereof is less than 0.2% by mass of total API, the process which includes a step of coupling related substance 1 to 2-phenylacetyl isothiocyanate. The present invention is even more preferably a process for producing a mesylic acid salt of compound 1 in which a content of each of related substance 1 and a salt thereof is less than 0.2% by mass of total API, the process in which a step of coupling related substance 1 to 2-phenylacetyl isothiocyanate is included and a content of related substance 1 charged in one production is 1 kg or more.

In another embodiment, the present invention is a process for producing compound 1 or a salt thereof, in which a content of each of related substance 2 and a salt thereof is less than 0.2% by mass of total API. The present invention is preferably a process for producing compound 1 or a salt thereof in which a content of each of related substance 2 and a salt thereof is less than 0.2% by mass of total API, the compound 1 or a salt thereof which is derived from methyl 4-chloro-7-methoxyquinoline-6-carboxylate, 2-fluoro-4-nitrophenol, and 2-phenylacetyl isothiocyanate. The present invention is more preferably a process for producing a mesylic acid salt of compound 1 in which a content of each of related substance 2 and a salt thereof is less than 0.2% by mass of total API, the process includes a step of coupling related substance 1 to 2-phenylacetyl isothiocyanate. The present invention is even more preferably a process for producing a mesylic acid salt of compound 1 in which a content of each of related substance 2 and a salt thereof is less than 0.2% by mass of total API, the process which a step of coupling related substance 1 to 2-phenylacetyl isothiocyanate is included and a content of related substance 1 charged in one production is 1 kg or more.

In another embodiment, the present invention is a process for producing compound 1 or a salt thereof, in which a content of each of related substances 3 and a salt thereof is less than 0.2% by mass of total API. The present invention is preferably a process for producing compound 1 or a salt thereof in which a content of each of related substance 3 and a salt thereof is less than 0.2% by mass of total API, the compound 1 or a salt thereof which is derived from methyl 4-chloro-7-methoxyquinoline-6-carboxylate, 2-fluoro-4-nitrophenol, and 2-phenylacetyl isothiocyanate. The present invention is more preferably a process for producing a mesylic acid salt of compound 1 in which a content of each of related substance 3 and a salt thereof is less than 0.2% by mass of total API, the process which includes a step of coupling related substance 1 to 2-phenylacetyl isothiocyanate. The present invention is even more preferably a process for producing a mesylic acid salt of compound 1 in which a content of each of related substance 3 and a salt thereof is less than 0.2% by mass of total API in which a step of coupling related substance 1 to 2-phenylacetyl isothiocyanate is included and a content of related substance 1 charged in one production is 1 kg or more.

In another embodiment, the present invention is a process for producing compound 1 or a salt thereof, in which a content of each of related substance 4 and a salt thereof is less than 0.2% by mass of total API. The present invention is preferably a process for producing compound 1 or a salt thereof in which a content of each of related substance 4 and a salt thereof is less than 0.2% by mass of total API, the compound 1 or a salt thereof which is derived from methyl 4-chloro-7-methoxyquinoline-6-carboxylate, 2-fluoro-4-nitrophenol, and 2-phenylacetyl isothiocyanate. The present invention is more preferably a process for producing a mesylic acid salt of compound 1 in which a content of each of related substance 4 and a salt thereof is less than 0.2% by mass of total API, the process which includes a step of coupling related substance 1 to 2-phenylacetyl isothiocyanate. The present invention is even more preferably a process for producing a mesylic acid salt of compound 1 in which a content of each of related substance 4 and a salt thereof is less than 0.2% by mass of total API, the process in which a step of coupling related substance 1 to 2-phenylacetyl isothiocyanate is included and a content of related substance 1 charged in one production is 1 kg or more.

In another embodiment, the present invention is a process for producing compound 1 or a salt thereof, in which a content of each of related substances 5 and a salt thereof is less than 0.2% by mass of total API. The present invention is preferably a process for producing compound 1 or a salt thereof in which in which a content of each of related substance 5 and a salt thereof is less than 0.2% by mass of total API, the compound 1 or a salt thereof which is induced from methyl 4-chloro-7-methoxyquinoline-6-carboxylate, 2-fluoro-4-nitrophenol, and 2-phenylacetyl isothiocyanate. The present invention is more preferably a process for producing a mesylic acid salt of compound 1 in which a content of each of related substance 5 and a salt thereof is less than 0.2% by mass of total API, the process which includes a step of coupling related substance 1 to 2-phenylacetyl isothiocyanate. The present invention is even more preferably a process for producing a mesylic acid salt of compound 1 in which a content of each of related substance 5 and a salt thereof is less than 0.2% by mass of total API, the process in which a step of coupling related substance 1 to 2-phenylacetyl isothiocyanate is included and content of related substance 1 charged in one production is 1 kg or more.

One embodiment of the present invention is a process for producing a mesylic acid salt of compound 1, which includes a step of producing compound 1 by coupling related substance 1 to 2-phenylacetyl isothiocyanate, and a step of producing a mesylic acid salt from compound 1 obtained in the step described above.

Further, one embodiment of the present invention is a process for producing a mesylic acid salt of compound 1, which includes a step of producing related substance 1 from methyl 4-(4-amino-2-fluorophenoxy)-7-methoxyquinoline-6-carboxylate by aminolysis, a step of producing compound 1 by coupling related substance 1 obtained in the step described above to 2-phenylacetyl isothiocyanate, and a step of producing a mesylic acid salt from compound 1 obtained in the step described above.

Further, one embodiment of the present invention is a process for producing a mesylic acid salt of compound 1, which includes a step of producing compound 1 by coupling related substance 1 to 2-phenylacetyl isothiocyanate, a step of purifying compound 1 obtained in the step described above, and a step of producing a mesylic acid salt from compound 1 purified in the step described above.

Further, one embodiment of the present invention is a process for producing a mesylic acid salt of compound 1,which includes a step of producing related substance 1 from methyl 4-(4-amino-2-fluorophenoxy) -7-methoxyquinoline-6-carboxylate by aminolysis, a step of producing compound 1 by coupling related substance 1 obtained in the step described above to 2-phenylacetyl isothiocyanate, a step of purifying compound 1 obtained in the step described above, and a step of producing a mesylic acid salt from compound 1 purified in the step described above.

Further, one embodiment of the present invention is a process for purifying compound 1, which includes a step of recrystallizing compound 1.

Further, one embodiment of the present invention is a process for purifying compound 1, which includes a step of recrystallizing compound 1 using a solvent containing N,N-dimethylacetamide and ethanol.

### EXAMPLE

Hereinafter, the present invention will be described in more detail with reference to Examples and Comparative Examples, but the present invention is not limited to these Examples.

As various reagents used in Examples and Comparative Examples, commercially available products were used unless otherwise specified.

A content of related substance 1 contained in a free form or a mesylic acid salt of compound 1 was measured by high performance liquid chromatography.

### [Reference Example 1] Synthesis of Related Substance 1:

Related substance 1 (compound 46a of Patent Literature 1) was synthesized with reference to Example 46 of Patent Literature 1.

### [Example 1] Synthesis of Free Form of Compound 1 from Related Substance 1:

Under a nitrogen atmosphere, ethanol (114.6 mL) and related substance 1 (27.50 g) were charged to toluene (571.6 mL), and the mixture was stirred. After 2-phenylacetyl isothiocyanate (21.42 g) was added dropwise thereto, the mixture was stirred at an internal temperature of 20-30°C (target 25°C) for 20 hours. After confirming the completion of the reaction, the precipitate was collected by filtration and cleaned with toluene.

The precipitate was dried under reduced pressure at 40-50°C to obtain a free form of compound 1 (yield: 39.40 g, yield: 94.8%).

### [Comparative Example 1] Aminolysis of methyl 4-(2-fluoro-4-(3-(2-phenylacetyl)thioureido)phenoxy)-7-methoxyquinoline-6-carboxylate:

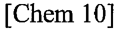

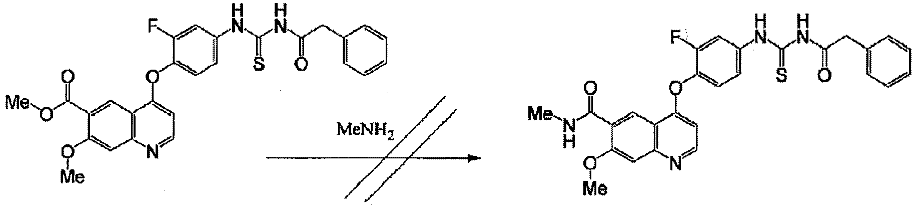

For the purpose of searching for an appropriate substrate for constructing methylamide from carboxylic acid or ester at the 6-position of quinoline, aminolysis using methylamine was attempted with respect to methyl 4-(2-fluoro-4-(3-(2-phenylacetyl)thioureido)phenoxy)-7-methoxyquinoline-6-carboxylate (hereinafter, also referred to as compound 2) as shown in the above scheme.

Compound 2 was synthesized using a compound such as a compound 39b of Patent Literature 1 instead of related substance 1 as in the conditions of Example 1.

However, even when aminolysis using methylamine was performed in compound 2, no compound 1 was obtained.

In addition, a method of hydrolyzing compound 2 to obtain a carboxylic acid and then condensing the carboxylic acid with methylamine was also studied. However, the target carboxylic acid was not obtained at the time of hydrolysis.

Therefore, it was determined that it was not suitable to obtain compound 1 after methylamide was constructed from compound 2.

### [Example 2] Recrystallization of compound 1 with Solvent which Contains N,N-dimethylacetamide and Ethanol (Purification Step 1) and Production of Mesylic Acid Salt (Step 2):

### (Step 1)

A free form (3 g) of compound 1 obtained in Example 1 was charged to N,N-dimethylacetamide (12 mL), and the temperature was raised to 75°C to dissolve compound 1. Ethanol (24 mL) was added dropwise thereto, and the mixture was stirred at 50°C for 30 minutes, then cooled to 5°C, and stirred for 1 hour for crystallization. Then, the precipitate was collected by filtration to obtain a free form (2.5 g) of compound 1.

### (Step 2)

With reference to Example 1 of Patent Literature 4, a mesylic acid salt of compound 1 was obtained from the free form of compound 1.

When the total content of related substances 1 and a salt thereof contained in the mesylic acid salt of compound 1 was measured, it was found that the total content of related substances 1 and the salt thereof was reduced to less than 0.05% by mass of total mesylic acid salt of compound 1. Therefore, it was determined that the purification of compound 1 shown in Example 1 by recrystallization (step 1) was suitable for the purification of an API.

### [Reference Example 2] Recrystallization of Free Form of Compound 1 by Dimethylsulfoxide (Purification step):

A free form of compound 1 obtained in Example 1 was charged into dimethyl sulfoxide and heated at 85°C. However, compound 1 tended to be decomposed, and purification by recrystallization was difficult.

### [Example 3] Production of Free Form of Compound 1:

### (Step 1) Synthesis of methyl 4-(4-nitro-2-fluorophenoxy)-7-methoxyquinoline-6-carboxylate

According to a method described in Example 39 (compound 39a) of Patent Literature 1, methyl 4-(4-nitro-2-fluorophenoxy)-7-methoxyquinoline-6-carboxylate was obtained from methyl 4-chloro-7-methoxyquinoline-6-carboxylate and 2-fluoro-4-nitrophenol.

### (Step 2) Synthesis of methyl 4-(4-amino-2-fluorophenoxy)-7-methoxyquinoline-6-carboxylate

Methyl 4-(4-nitro-2-fluorophenoxy)-7-methoxyquinoline-N-methylquinoline-6-carboxylate (7.00 g) obtained in step 1 and 1% carbon-supported platinum (Pt/C; 0.7 g) were charged to dimethoxyethane (DME; 70 mL), and the mixture was stirred. An operation of pressurizing to 0.2 to 0.3 MPa with nitrogen gas and discharging the pressure was performed three times, and then an operation of pressurizing to 0.2 to 0.3 MPa with hydrogen gas and discharging the pressure was performed three times. The mixture was pressurized to 0.2 to 0.3 MPa with hydrogen gas, heated to an internal temperature of 70 ± 5°C, and then stirred at the same temperature for 2 hours or more. After confirming the completion of the reaction, the mixture was cooled to an internal temperature of 40 ± 5°C. The reaction solution was filtered at the same temperature and then cleaned with DME. The filtrate was concentrated under reduced pressure to 70 mL, the internal temperature was adjusted to 25 ± 5°C, and then water (105 mL) was added dropwise over 30 minutes or more. After the dropwise addition, the mixture was stirred at the same temperature for 2 hours or more. After crystals were collected by filtration, the crystals were cleaned with DME/water (1/4) (35 mL). The obtained crystals were dried under reduced pressure at an external temperature of 45 ± 5°C to obtain methyl 4-(4-amino-2-fluorophenoxy)-7-methoxyquinoline-6-carboxylate (6.53 g, yield 101.5%).

### (Step 3) Synthesis of Related Substance 1

According to a method described in Example 46 (46a) of Patent Literature 1, related substance 1 was obtained from methyl 4-(4-amino-2-fluorophenoxy)-7-methoxyquinoline-6-carboxylate obtained in step 2.

### (Step 4)

Under a nitrogen atmosphere, ethanol (95.75 L) and related substance 1 (22.96 kg) were charged to toluene (477.1 L), and the mixture was stirred. Then, 2-phenylacetyl isothiocyanate (pure amount: 17.88 kg) was added dropwise thereto, followed by stirring at an internal temperature of 20 to 30°C (target: 25 °C) for 6 hours. After confirming the completion of the reaction, the mixture was further stirred at the same temperature for 1 hour, and the precipitate was collected by filtration and cleaned with toluene.

The precipitate was dried under reduced pressure at 40-50°C to obtain a free form of compound 1 (yield: 34.22 kg, yield: 98.1%).

Since the producing process had a small number of steps and compound 1 having a small amount of impurities can be obtained without purification by silica gel column chromatography, it was determined that the quality of medicines can be maintained and the producing process is suitable for mass production.

### [Example 4] Mass Production of Mesylic Acid Salt of Compound 1:

With reference to Example 1 of Patent Literature 4, a mesylic acid salt of compound 1 was obtained from a free form (11.95 kg) of compound 1 obtained in Example 3 (yield: 11.50 kg, yield: 81.2%). A content of each of related substances 1 to 5 or salts thereof with respect to the mesylic acid salt of compound 1 was less than 0.2% by mass.

Since the producing process had a small number of steps as in Example 3, and a mesylic acid salt of compound 1 having a small amount of impurities can be obtained without purification by silica gel column chromatography, it was determined that the quality of medicines can be maintained and the producing process is suitable for mass production.

Although the present invention has been described in detail and with reference to particular embodiments, it will be apparent to those skilled in the art that various changes and modifications can be made without departing from the spirit and scope of the invention. This application is based on Japanese Patent Application No. 2020-023754 filed on February 14, 2020, Japanese Patent Application No. 2020-086940 filed on May 18, 2020, Japanese Patent Application No. 2020-157815 filed on September 18, 2020, and Japanese Patent Application No. 2020-157816 filed on September 18, 2020, the contents of which are incorporated herein by reference.

## Claims

1. A process for producing 4-(2-fluoro-4-(3-(2-phenylacetyl)thioureido)phenoxy)-7-methoxy-N-methylquinoline-6-carboxamide, comprising:
coupling 4-(4-amino-2-fluorophenoxy)-7-methoxy-N-methylquinoline-6-carboxamide to 2-phenylacetyl isothiocyanate.

2. The producing process according to claim 1, comprising:
obtaining the 4-(4-amino-2-fluorophenoxy)-7-methoxy-N-methylquinoline-6-carboxamide from methyl 4-(4-amino-2-fluorophenoxy)-7-methoxyquinoline-6-carboxylate by aminolysis.

3. The producing process according to claim 1 or 2, comprising:
deriving the 2-phenylacetyl isothiocyanate from 2-phenylacetyl chloride.

4. The producing process according to any one of claims 1 to 3, wherein a solvent which contains toluene and ethanol is used in the coupling step.

5. A process for producing a mesylic acid salt of 4-(2-fluoro-4-(3-(2-phenylacetyl)thioureido)phenoxy)-7-methoxy-N-methylquinoline-6-carboxamide, comprising:
producing the mesylic acid salt from 4-(2-fluoro-4-(3-(2-phenylacetyl)thioureido)phenoxy)-7-methoxy-N-methylquinoline-6-carboxamide obtained by the producing process according to any one of claims 1 to 4.
